# EUROPEAN PATENT APPLICATION

(11) **EP 1 550 731 A1**
(43) Date of publication of application: **06.07.2005**
(21) Application number: 04030895.9
(22) Date of filing: 28.12.2004
(51) Int. Cl.: C12Q 1/68

(54) **A method for quantitative determination of multi-drug resistance in tumours**

(30) Priority: 30.12.2003 US 748713
(71) Applicant: Eppendorf Array Technologies SA, 5000 Namur (BE)
(72) Inventor: Remacle, Jose, 5020 Malonne (BE); Efferth, Thomas, 69121 Heidelberg (DE); Gillett, Jean-Pierre, 6870 Saint-Hubert (BE)
(74) Representative: Becker Kurig Straus

(57) **Abstract**

The invention relates to a method for determining, whether a cell of interest is resistant to an active substance to which it is exposed, e.g. during cancer chemotherapy. The method comprises the quantitative analysis of the expression of at least 5 ABC transporter genes within a sample, treated in the same manner using a DNA micro-array.

## Description

### FIELD OF THE INVENTION

The present invention relates to a method for monitoring the development of cell resistance to active substances to which they are exposed, e.g. during cancer chemotherapy. The method comprises the quantitative analysis of the expression of at least 5 ABC transporter genes within a sample, treated in the same manner using a DNA micro-array.

The determination of the gene expression pattern of at least 5 ABC transporter genes brings a solution to the problem of development of resistance in cells upon the action of a drug. The determined pattern of expression allows (I) to unravel multi-drug resistance functions, (II) to reliably correlate the resistance of cells from a patient to the chemotherapy by a given drug, (III) to determine a drug active against the cell or cell type of interest, (IV) to select an active drug for patient treatment and (V) to monitor a patient treated with a drug for chemotherapy. The method is particularly well suited for the screening of ABC transporter genes in acute myeloid leukemia or acute lymphocytic leukemia or solid tumors for which there is a frequent relapse due to the expression of these genes.

### BACKGROUND OF INVENTION

The development of multi-drug resistance (MDR) is supposed to be a major cause of failure of cancer chemotherapy, which resistance seems to be mediated by a variety of different cellular mechanisms, inter alia the cycle stage of the tumor cells, the detoxification mechanisms, the cellular drug transport and the DNA replication and repair mechanisms.

The MDR phenotype is characterized by resistance to a broad spectrum of structurally unrelated cytotoxic drugs. Cellular mechanisms known to be involved in the development of cellular resistance included the topoisomerase II (Beck et al., Adv. Enz. Regul. **33** (1993), 113), drug detoxification enzymes, such as the gluthatione-S-transferases (Morrow and Cowan, Cancer Cells **2** (1990), 15) and alterations in drug-induced apoptosis, involving genes in the Bcl-2 pathway (Reed, Hematol./Oncol. Clin. N. Amer. **9** (1995), 451). These cellular alterations are a particular limitation to cancer chemotherapy, with the MDR cell often displaying other properties in addition, such as genome instability and loss of checkpoint control, that complicate further therapy. MDR may be intrinsic (before exposure to chemotherapeutic agents) or may be acquired after chemotherapy.

Also, the over-expression of some ATP binding cassette (ABC) transporters has been linked with MDR (van den Heuvel-Eibrink et al., Int. J. Clin. Pharm. and Ther. **38** (2000), 94-110). The ABC proteins itself comprise a superfamily of membrane ATP-dependent active transporters that share a common ATP binding motif (Schriml and Dean, Genomics **64** (1999), 24-31; Demolombe and Escande, Trends Pharmacol. Sci. **17** (1996), 273-275). Structurally, they are composed of four structural domains, two transmembrane domains and two ATP-hydrolyzing domains (Schneider and Hunke, FEMS Microbiol. Rev. **22** (1998), 1-20). The transmembrane domains, each consisting of six membrane-spanning helices, form the pathway through which substrates cross the membrane. The ATP-hydrolyzing domains are located at the cytosolic face of the membrane and comprise Walker A and B motifs, which are conserved among ABC transporters, regardless of the substrate specificity or species of origin of a particular ABC transporter. The substrate specificity of a particular transporter is ultimately determined by the transmembrane domains.

The endogenous task of these proteins is to transport a wide variety of different molecules across cell membranes, including amino acids, nucleotides, sugars, lipids, peptides. They play roles in signal transduction, protein secretion, antigen presentation, and bacterial pathogenesis, nutrient uptake, and sporulation. Some also regulate the activities of other ion channels. Besides their presence in plasma membranes, ABC transporters are found in the membranes of intracellular organelles such as the endoplasmic reticulum, vacuoles, peroxisomes, and mitochondria.

At present, the human superfamily of ABC transporters comprises 48 characterized genes organized in 7 subfamilies. Details of the nomenclature scheme can be found at: http://www.gene.ucl.ac.uk/nomenclature/genefamily/abc.html, which document is incorporated herein by way of reference. The homology between those genes is high and for some of them higher than 70%.

Inhibitors of the major ABC genes contributing to MDR have been developed, and extensive experimentation and clinical research have been performed to attempt to block the development of drug resistance during chemotherapy. In this respect WO 01/36477 discloses to inhibit the biological activities of ABC transporters by exposing the cells to peptides which interfere with correct transporter assembly. WO 00/26245 proposes antagonists of ABC proteins for administration to a subject to be treated, with the antagonist being an antibody which specifically binds ABC proteins.

Also a variety of studies for the diagnosis o MDR focused on ABC transporter genes, in particular on MDR1/ABCB1 and MRP1/ABCC1. Several tools have been developed for diagnosis of MDR1 gene expression in clinical samples: (a) gene probes or primers allowing the evaluation of MDR1 gene expression; (b) antibodies allowing the detection of P-glycoprotein, and (c) dyes for functional studies of P-glycoprotein mediated drug efflux (Robert, Eur. J. of Clin. Invest. **29** (1999), 536-545; Efferth, Leukemia **13** (1999), 1627-1629). For gene expression, described methods comprises Northern blots and RT-PCR at the tissue level and in situ hybridization at the cellular level. Schwarzenbach proposes a multiplex RT-PCR in combination with nested PCR for quantitative analysis of mRNA expression of MDR1 and MRP (Ann. Biochem. **308** (2002), 26-33). Recently, a quantitative real-time PCR determination of MDR1 gene expression have been validated in 101 patients with acute myeloid leukemia (Olesen et al Eur. J. Haematol. **70** (2003), 296-303).

However, all these methods are limited to only one or a few of the MDR proteins or genes did not proof suitable for monitoring MDR in a cell/a sample.

Another way to determine the development of resistance in a cell was proposed in US 2001/0019846, wherein a functional method for quantitative in vitro determination of MDR cells is proposed. The method is based on the measurement of the accumulation rate of free calcein within the cells of the specimen, after exposing the cells in vitro to a cell permeable form of the calcein that is good substrate for MDR proteins present in the sample. However, also this method did not provide an overall assessment of the development of MDR in a cell.

Another problem in dealing with the analysis of clinical material is its unpredictability. One is never sure at which time the sample will be available and the condition in which it will arrive (Kristan and Arya, Hematol. Oncol. Clin. N. Am. **16** (2002), 357-372). Considering the number of steps and personnel involved in moving a specimen from the patient to the laboratory and then its analysis, there is a high need to standardize the diagnostic protocol especially to control the storage and extraction of the sample and to include adequate controls that can be used to validate data on the clinical sample.

At present, the use of MDR diagnosis for treatment of human cancer appears premature for the following reasons: (i) there is no agreed and uniform technique for the diagnosis of MDR, (ii) other mechanisms of resistance to any of the MDR drugs exist in tumors concurrently with MDR1 and contribute to clinical resistance. Despite intensive research efforts, both at the preclinical work and clinical trials, there is no definitive evidence that the diagnosis of MDR1 alone and modulation are of value in cancer treatment. These failures could point to the multi-factorial nature of drug resistance in refractory cells. So far, most of the studies were focused on the expression of the well characterized MDR1/ABCB1 and MRP1/ABCC1 genes. However the expression of these two genes by themselves is not sufficient for a correlation with clinical data.

There is a need for a reliable tool for determining MDR in a sample, including other makers to obtain data which would be relevant to explain the clinical resistance of cells to drug treatment.

High density DNA microarray assays have been used to identify new markers associated with phenotype resistance to Doxorubicin (Watts et al., J Pharmacol. Exp. Ther. **299** (2001), 434-441). Tumor cell resistance to doxorubicin is known to be often associated with the expression of the ABCB1 gene. The authors effectively found the ABCB1 gene to be over-expressed in the resistant cells. They also found changes in the genes associated with apoptosis. The limitation of high density micro-arrays is the loose quantification of the genes and they mostly detect the highly differentiated expressed genes.

The invention proposes a new method for the determination of the presence of MDR based on the determination of the expression profile of at least 5 ABC genes on low density quantitative micro-array.

The array considered may comprises essentially all members of the ABC superfamily including the 3 main ABC genes (ABCB1, ABCC1 and ABCG2) known to be involved in multi-drug resistance. The function of most of the ABC genes remain to be unravelled so far. It is only recently that some ABC transporters have been shown to be involved in drug resistance (Table 3). The present invention provides for the first time a tool which will allow to unravel the function of thus far unknown ABC transporters in the development multi-drug resistance.

The method allows the simultaneous quantification of all ABC transporters in the same sample, treated in the same manner. The method is also well suited to obtain a reliable diagnosis of the multi-drug resistance phenotype based on the expression of a few well characterized genes. The method is also suitable to analyze the function of unkown ABC transporters in correlation with the multi-drug resistance phenotype. It is also of primarily interest to correlate the expression pattern of the ABC transporters with clinical data.

### Definitions

In the context of the present application and invention the following definitions apply :
The term "expressed genes" shall designate the parts of the genomic DNA which are transcribed into mRNA and then translated into a peptides or proteins. The measurement of the expressed genes is performed on either molecules within this process most currently the detection of the mRNA or of the peptide or protein. The detection can also be based on the specific property of the protein being for example its enzymatic activity.

The terms "nucleic acid, array, probe, target nucleic acid, bind substantially, hybridizing specifically to, background, quantifying" are as described in the international patent application WO97/27317, which is incorporated herein by way of reference.

The term "nucleotide triphosphate" refers to nucleotides present in either as DNA or RNA and thus includes nucleotides which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "nucleotide" as used herein refers to nucleosides present in nucleic acids (either DNA or RNA) compared with the bases of said nucleic acid, and includes nucleotides comprising usual or modified bases as above described.

References to nucleotide(s), polynucleotide(s) and the like include analogous species wherein the sugar-phosphate backbone is modified and/or replaced, provided that its hybridization properties are not destroyed. By way of example the backbone may be replaced by an equivalent synthetic peptide, called Peptide Nucleic Acid (PNA).

The terms "nucleotide species" is a composition of related nucleotides for the detection of a given sequence by base pairing hybridization; nucleotide species are from the same family of closely related sequences which will hybridized with one specific target sequence, the non specific hybridization being not significant or as low so as not to interfere with the quantification of the specific target. Typically capture probe species are one oligonucleotide sequence synthesized chemically and having side products arising from the fact that the synthesis is not obtained at 100% so that some low amount of related sequences are present within the main sequence. Also, polynucleotides sequences synthesized by enzymatic amplification may contain some mis-incorporation of nucleotides but in general at such a low level that it does not interfere with the capture probe's task to serve as a specific capture of the target. The essential characteristic of a nucleotides species for the invention is that the overall species can be used for capture of a given sequence belonging to a given gene.

The term "polynucleotide" sequences that are complementary to one or more of the genes described herein, refers to polynucleotides that are capable of hybridizing under stringent conditions to at least part of the nucleotide sequence of said genes. Polynucleotides also include oligonucleotides being below 100 bases which can be used under particular conditions. Such hybridizable polynucleotides will typically exhibit at least about 75% sequence identity at the nucleotide level to said genes, preferably about 80% or 85% sequence identity or more preferably about 90% or 95% or more nucleotide sequence identity to said genes. They are composed of either small sequences typically 15-30 base long or longer ones being between 30 and 100 or even longer between 100 and 800 base long.

The term "bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The term "capture probe" designates a molecule which is able to specifically bind to a given polynucleotide or polypeptide. Polynucleotides binding is achieved via base pairing between two polynucleotides, one being the immobilized capture probe and the other one the target to be detected. Polypeptide binding is best performed using antibodies specific for the polypeptide for the capture of a given polypeptide or protein. Part of the antibodies, or recombinant proteins incorporating part of the antibodies, typically the variable domains, or even proteins being able to specifically recognized the peptide can also be used as capture probes.

The terms "background" or "background signal intensity" refers to hybridization signals resulting from non-specific binding, or other interactions, between the labelled target nucleic acids and components of the polynucleotide array (e. g. the polynucleotide probes, control probes, the array substrate, etc.). Background signals may also be produced by intrinsic fluorescence of the array components themselves. A single background signal can be calculated for the entire array, or a different background signal may be calculated for each target nucleic acid. In a preferred embodiment, the background is calculated individually for each spot, being the level intensity of the signal around each of the spot.

The phrase "hybridizing specifically to" refers to the binding, duplexing or hybridizing of a molecule substantially to or only to a particular nucleotide sequence or sequences under stringent conditions when that sequence is present in a complex mixture (e. g., total cellular) DNA or RNA. Specific detection and quantification means a detection which is specific for a given gene or protein, contamination or cross-reaction being below 5% and better below 1% and even better below 0.1 %.

The "hybridized nucleic acids" are typically detected by detecting one or more "labels" attached to the sample nucleic acids. The labels may be incorporated by any of a number of means well known to those of skill in the art, such as detailed in WO 99/32660, which is incorporated herein by way of reference.

The term "capture probes" in the sense of the present invention shall designate genes or parts of genes of different length, e.g. between 10 and 1500 nucleotides, which are either synthesized chemically in situ on the surface of the support or laid down thereon. Moreover, this term shall also designate polypeptides or fragments thereof, or antibodies directed to particular polypeptides, which terms are used interchangeably, attached or adsorbed on the support.

The term "homology" is intended to mean the degree of identity of one polynucleotide to another polynucleotide. According to the invention the term homology is used in connection with complementarity between polynucleotides within a family. There may be complete homology (i. e. 100% identity) between two or more polynucleotides. The degree of homology may be determined by any method well known for a person skilled in the art.

The term "sample" or "biological sample" includes within its meaning organisms, organs, tissues, cells derived from a patient or biological material produced by a cell culture. The biological sample may be living or dead and includes sample material directly exposed to the active substance, but also the progeny of the sample material, such as cells, tissues organs, that has been exposed to the active substance. The sample may correspond to one or more cells from the organisms, in case the organism is a multicellular organism, the sample may correspond to one or more cells from one or more tissues creating the multicellular organism. The biological sample to be used according to the invention may be derived from particular organs or tissues of the multicellular organism, or from isolated cells obtained from a single or multicellular organism. In obtaining the RNA's to be analyzed from the biological sample from which it is derived, the biological sample may be subject to a number of different processing steps. Such steps might include tissue homogenization, cell isolation and cytoplasma extraction, nucleic acid extraction and the like and such processing steps are generally well known for a person skilled in the art. Methods of isolating RNA from cells, tissues, organs or whole organisms are known to those skilled in the art and are described in e. g. Sambrook et al., Molecular Cloning : A Laboratory Manual, Cold Spring Harbor Press (1989). The biological sample may be of the same kind i. e. the biological sample is of the same kind of origin, such as coming from the same type of tissue, the same organism or the same type of organism or the same cell type etc.

The term "tissue" shall mean a collection of differentiated cells such as adrenal gland, total brain, liver, heart, kidney, lung, pancreas, mammary gland, placenta, prostate, salivary gland, skeletal muscle, small intestine, spleen, stomach, testis, thymus, trachea, and uterus.

The term "target polynucleotide" is intended to mean a polynucleotide present in the biological material of interest. The target polynucleotide encodes a polypeptide, which is at least a part of a amine receptor. If the target polynucleotide has a complementary polynucleotide present on the array, it will hybridize thereto and thus give rise to a detectable signal.

The term "multi-drug resistance" denotes the occurrence of transport protein mediated cell efflux in a cell. While such cell efflux typically transports a variety of substances to the outside of the cell membrane, such transport is particularly problematic in tumor cells, where it interferes with therapeutic treatment of cancer due to the active transport of a wide variety cytotoxic and cytostatic agents of significantly different structure to the exterior of the cell membrane. Such tumor cells are called "multi-drug resistant" cells.

The term "active substance" is intended to mean any agent or substance which has an impact on biological systems, such as cells. Examples for such agents or substances are chemotherapeutica.

### Brief description of the drawings and tables

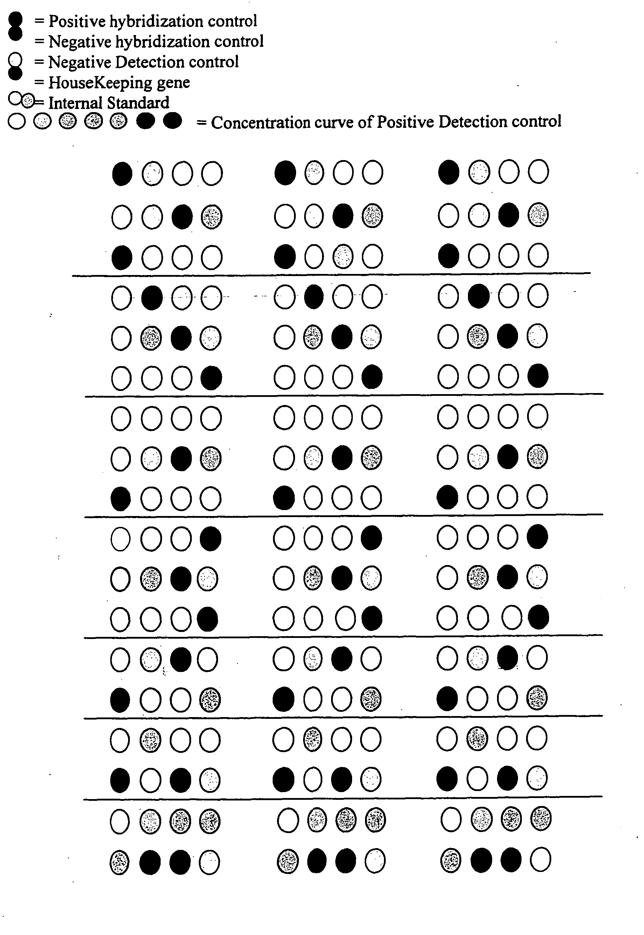

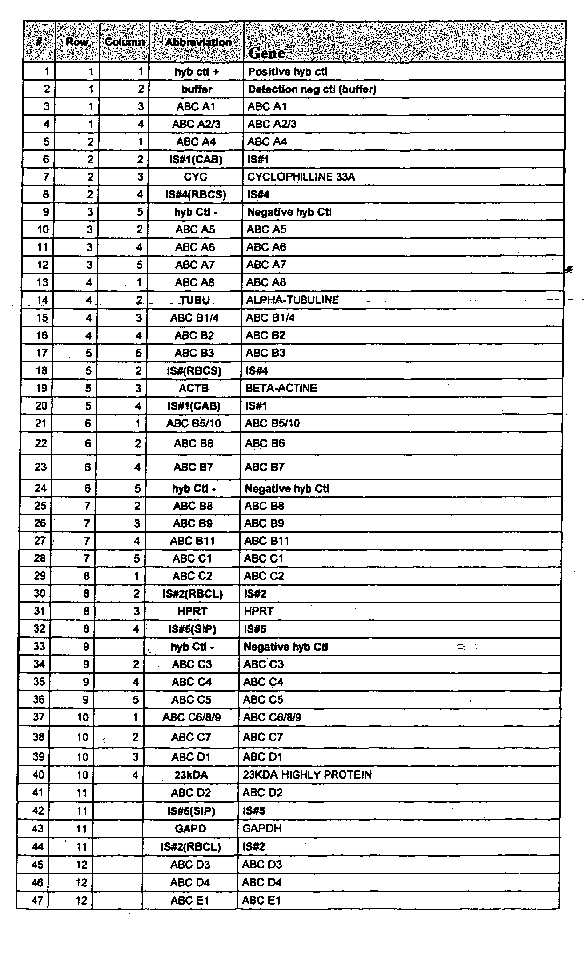

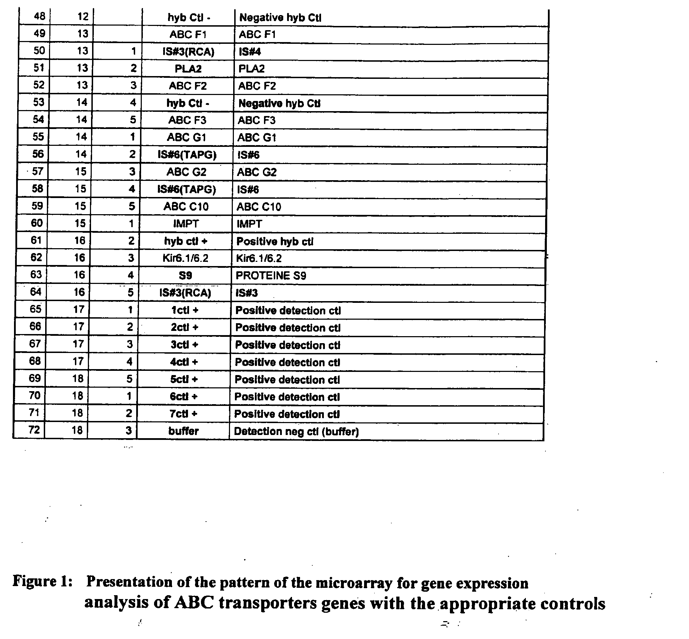

Fig. 1 shows the micro-array design (including the gene list) for monitoring gene expression of 42 different transporters by single analysis of the data present on the array pattern.
Table 1 lists ABC transporter families, which the skilled person may derive from http://www.gene.ucl.ac.uk/nomenclature/genefamily/abc.html.
Table 2 lists the ABC gene families in some characterized eukaryotes.
Table 3 presents the ABC genes known to be involved in the development of Drug Resistance, substrates and inhibitors.
Table 4 provides results obtained according to the method described in the invention for the determination of differentially expressed ABC transporters in drug-resistant sublines versus drug-sensitive parental cell line (example 1 : CCRF-ADR5000 vs CCRF-CEM; example 2 : HL60A/R vs HL60 sens; example 3 : MCF7-CH1000 vs MCF7 parental).
Table 5 provides results obtained according to the method described in invention for the determination of basal expression of the ABC transporters in drug―sensitive leukemia cell lines (CCRF-CEM; HL60 sens; MCF7 parental).

### DETAILED DESCRIPTION OF THE INVENTION

The resistance to chemotherapy is multi-factorial and may be mediated by different cellular mechanisms including the ABC transporters.

So far, only the expression of a few highly activated and over-expressed ABC transporter genes has been monitored, which is not sufficient to reliably determine MDR, and as a result of this to select an adapted cancer treatment.

The invention proposes a new method for the determination of the resistance of cells upon the action of a substance, such as a drug. The method is characterized by the simultaneous analysis of the gene expression pattern of said cells on a microarray which contains on specific locations thereon capture probes specific for the detection and/or quantification of at least 5 ABC transporters.

Thus, in case at least 5 ABC transporter gene exhibit an increased transcription/expression profile, a reliable correlation with the development of resistance in cells to a given substance may be derived.

Due to the publication of the human genome, essentially all of the sequences for human ABC genes are known and publicly available, e.g. from public databases, such as the National Center for Biotechnology Information (NCBI) or the LocusLink web site (http://www.ncbi.nlm.nih.gov/LocusLink/).

The web site (http://www.gene.ucl.ac.uk/nomenclature/genefamily/abc.html) provides an official nomenclature for human ABC transporter genes, aliases, subfamilies, chromosome location and sequence accessions. For other species many ABC transporter sequences are known and information stored in LocusLink web site. From these sequences, a skilled person may design appropriate capture probes for the specific detection of the gene nucleotide sequences.

Specific examples for different ABC transporters to be detected and analyzed in a single assay are presented in Table 1 and a preferred array/composition of the tool according to the present invention is shown in figure 1. Consequently, according to a preferred embodiment the ABC transporters to be investigated on the micro-array is at least 5, at least 10, at least 20, at least 39 and 49, selected from those listed in Table 1.

The present method and tool allows the specific determination of the expression pattern in one assay of at least 5 of the various ABC genes, as exemplified by the list provided in Table 1. This represents 5-49 genes for human, 5-45 genes for mouse, 5-39 genes for at, 5-56 genes for Drosophila, 5-58 genes for C. elegans and 5-31 genes for yeast as exemplified by the list provided in Table 2 for different species.

According to an embodiment of the invention, the variations of the gene expression fulfil the following criteria in order to be relevant for interpretation :
1) These variations are recognized as being, or at least suspected to be, of biological relevance for understanding the mechanism of cell resistance upon the action of a drug but they are also of diagnostic, prognostic, or predictive value.
2) They are expressed in cells at levels detectable by the DNA micro-array. The present invention gives also a means of providing micro-array with a direct detection of 39 ABC transporters, 1 cationic transporter and 2 ATP-sensitive potassium channels (figure 1) with cDNA prepared from as low as 1 to 10 µg of total RNA.
3) Variations of mRNA amount is parallel to variations of the corresponding protein and gene expression. The amount of mRNA at a given moment in the cells reflects the equilibrium between transcription and degradation. This amount is also influenced by gene deletions and amplifications. Protein structures and activities are the ultimate support of cell functions and tumor properties. A good correlation between mRNA and protein levels may be crucial when it concerns these therapeutic target proteins, The polynucleotide detection is favorably replaced by the polypeptide detection in order to directly obtain the quantification of the transcript of the genes.

In a preferred embodiment, the tool for the analysis of the ABC transporters is a microarray solid support on which are present capture molecules being single stranded DNA attached on specific locations, each location being covered by one species of capture probe for the detection and quantification of one subfamily or closely related subfamilies.

In the present invention, one spot is sufficient for identifying one gene. The direct relationship between the spot identification and the subfamily identification makes the invention particularly useful as a tool for screening the subfamilies in different samples since the overview of the results on the array directly corresponds to the overview of the subfamilies expression in the sample or in the tissue.

In principle, the micro-array may contain as few as 5 capture probes, i.e. one capture probe associated with each of 5 different subfamilies of ABC transporters. Yet, the number of capture probes on the micro-array may be selected according to the need of the skilled person and may contain capture probes for the detection of up to about 3000 different genes, e.g. about 100, or 200 or 500 or 1000, or 2000 different genes being either other subfamilies of ABC genes or other genes.

Beside the ABC transporters, the micro-arrays may also contain capture probes derived from other transport genes, like Kir6.1 and Kir6.2 that form channels with ABCC8 and ABCC9. Also, controls required for a quantification of the genes may be present. These include negative and positive controls, internal standards and house keeping genes. Exemplary results obtained with such a tool for the determination of the differentially expressed human ABC genes in resistant versus sensitive cell lines are presented in Table 4. The results were obtained on human cell lines since the capture probes present on the array were specific of human genes. Similar array may be constructed for other species including mouse, rat, drosophila, C. elegans and yeast with some adaptation in the sequence of the capture probes.

The capture probes may be any nucleotide which hybridize under given conditions to the probe molecule. In general, the capture probes may be of from about 15 to about 1000 nucleotides in length, preferably of from about 15 to about 400, or 15 to 200 nucleotides, or more preferred of from about 15 to about 100, or from 15 to 50, or from 15 to 30, and are fixed on a support being any solid support as long as they are able to hybridize with their corresponding cDNA and be identified and quantified. The density of the capture nucleotide sequences bound to the surface of the solid support is preferably above to 3 fmoles per cm² of solid support surface.

In a preferred embodiment of the invention, the low density micro-arrays allow the detection of changes in the ABC gene expression level occurring in cells with a significant change being observed when the gene expression of the tested cells differs by a factor of at least 1.5, preferably greater than 2, more preferably greater than 3 as compared to a reference.

Quantitative analysis of the gene expression is a requirement for the determination of the gene expression pattern of the ABC transporters and for the determination of the resistance of the cells to some drugs. Indeed, many of the ABC transporters genes in most of the cells are expressed at a basal level, as exemplified in table 5. Therefore, the assay has to be sufficiently sensitive and reliable to detect low significant changes in the expression of these genes (low gene induction or repression) since it will influence the resistance of the cells to some drugs. The increase or decrease of gene expression compared has to be analyzed in a quantitative way on all genes to be detected on the array in order to be able to draw significant conclusion on the resistance of the analyzed cells toward one or many drugs. The quantification of the results on the gene expression in the sample microarray is compared to a reference being either a reference material (e.g. cellular material that has not been exposed to the active substance), a reference array or a computation of data obtained on arrays which allows the comparison of sample to sample and the determination of significant changes in gene expression in the analyzed sample.

In a particular embodiment, the gene expression of the gene of interest for this invention is related to the presence of other genes present in the sample which are chosen as reference genes. Such genes are typically house keeping genes. They allow comparison of the quantification of a given gene in a sample compared to another sample and the detection of a potential increase of the expression of a gene of interest.

The final information requirement for the patient from which the cells are analyzed is to know if the cells are resistant to a particular drug and/or which are the drugs the cells will be sensitive to. For this purpose, the knowledge on the expression of many ABC transporters have to be obtained on a quantitative basis.

The nucleic acid to be assayed/investigated is either RNA from cells of interest or cDNA obtained therefrom, the latter of which is easier to handle. The cDNA may be obtained by retro-transcription from total RNA or mRNA. To this end, total RNA is extracted from tissue in an amount of about 0.1 to 100 µg, preferably about 0.1. to 50 µg, more preferably about 0.1. to 20 µg, even more preferably about 0.1 to 10 µg, or even more preferred between about 0.1 and 2 µg and is directly used for hybridization on the array. mRNA may also be processed in the same way with a much lower amount to be used for the copying into cDNA.

When RNA is amplified by means of a T7 polymerase based method, PCR, rolling circle or other methods, detection is possible even at a concentration lower than 0.1 µg of total RNA or mRNA depending on the amplification obtained being usually in the order of a few hundreds for the T7 polymerase and much higher for the PCR or rolling circle amplifications. In extreme cases, the investigation of a single cell or a group of a few cells, such as obtained by laser dissection methods is feasible. In amplification methods, however, different genes are amplified with different efficiencies and corrections may have to be provided.

According to another preferred embodiment, the original nucleotide sequences to be detected and/or to be quantified are RNA sequences copied by the retro-transcription of the 3' or 5' end wherein consensus primer and possibly also a stopper sequence are used. Preferably, the copied or amplified sequences are detected without previous cutting of original sequences into smaller portions.

When preparing cDNA, the DNA may at the same time be labeled, e.g. by including in the enzymatic process nucleotides harboring an appropriate label. Alternatively, a label may be attached to the nucleic acid, including, for example, nick translation or end-labeling by attachment of a nucleic acid linker joining the sample nucleic acid to a label. In an alternative embodiment, also the capture probes attached to the support may be labeled.

Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., cyanine dyes, such as Cy5, fluorescein, texas red, rhodamine, green fluorescent protein, and the like), radiolabels (e.g., ³H, ¹²⁵I, ³⁵S, ¹⁴C, or ³²P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Patent Nos. 3,817,837; 3,850,752; 3,939,350; 3,996,345; 4,277,437; 4,275,149; and 4,366,241.

Also, variants of the genes may specifically be detected and quantified on the array. In some tissues, multiple mRNAs are transcribed from the same gene. These variants often exhibit more or less overlapping sequences. Selection of the capture probes has to be specific for such marker, when all variants do not have the same potential importance as diagnostic, prognostic, or predictive indicator, or when it is recommended to detect only some specific ones.

Transport subfamilies are sometimes closely related so that the corresponding capture probes may be designed to enable differentiation between these closely related subfamilies or to recognize several subfamilies. For example a capture probe may be designed common for subfamilies ABCA2/ABCA3, ABCB1/ABCB4, ABCB5/ABCB10, ABCC6/ABCC8/ABCC9, Kir 6.1/ Kir 6.2 and giving an overall analysis of these subfamilies, while capture probes, distinguishing between the other subfamilies may be designed, which are e.g. shown in figure 1.

The target nucleic acid is contacted with the micro-array under conditions allowing hybridization of complementary strands only. Hybridization processes and conditions are well known in the art and are described e.g. in Sambrook et al., Molecular Cloning: A Laboratory Manual, 2^{nd} Ed., Cold Spring Harbor Laboratory Press, Cold Spring Harbor, N.Y. (1989).

E.g. the hybridization step may be performed under a cover glass in hybridization chambers or in a hybridization oven which essentially represent a diffusion limited process. Altematively, the reaction may be carried out with agitation, which decrease the required hybridization time. When using hybridization involving movement of the micro-array, the hybridization time may be reduced to a period of 60 minutes or less, preferably to 30 minutes or less. As a result, the total assay can be performed in less than 4, more preferably 3.5 hours.

After hybridization with the target nucleotides of the biological sample the spot intensity is read according to the label utilized, e.g. in fluorescence or colorimetry. The quantification of the genes may be performed by standard techniques, e.g. by comparison with internal standards introduced in the retro-transcription of mRNA.

In case the target nucleic acid has been labeled prior to contacting it with the micro-array, the signal and/or the signal intensity is a means for determining the activation of a particular ABC transporter or a change thereof.

The support as such may be made from any material conventionally used for this purpose and is preferably selected from the group consisting of glasses, electronic devices, silicon supports, silica, metal or mixtures thereof prepared in format selected from the group of slides, discs, gel layers and/or beads.

Apart from collecting information about the resistance condition of a cell collected from a patient subjected to a chemical therapy, e.g. antibiotics or chemotherapy, the present method also allows to give information about the potential of new active substances to provoke the development of resistance in a cell. Therefore, potential new drugs may be screened to determine, if the application of the drug alters the expression of the genes referred to herein. Expression being induction or repression of gene content of the cell, the most important being the induction of gene associated with the resistance mechanism.

This may be useful, for example, in determining whether a particular drug is effective in treating a multidrug resistance patient. In the case where a gene expression of the patient cells is not affected by the potential drug such that its level of expression is normal or stays normal for a long period of time, the drug is indicated in the patient treatment Similarly, a drug which causes over-expression of a multi-drug resistant transporter gene, may be contraindicated in the treatment of the disease. Cells are incubated with the drug for some periods of time being a few hours to several days and the pattern of gene expression is determined according to the present invention.

Specifically, a gene expression analysis may be performed on the present micro-array by contacting the nucleic acid derived from cells or tissues that had been subject to a particular environmental situation, e.g. had been incubated in the presence of (potential) drugs. Then, the data about the expression of the genes investigated are compared with the expression profile either from cells or tissues that had not been subjected to a particular environmental situation.

Based on the array identification of gene expression, the effect of the drug or chemical may then be evaluated for their potential activity. Therefore, the present invention also comprises a method for using the present micro-array in the diagnostic of multi-drug resistance in the analysed cell, selection of a drug active against the analyzed cell and monitoring patient treated with a drug for chemotherapy which are associated with the over-expression of several ABC transporter genes.

Assays to monitor the expression of a marker or markers, as e.g. defined in table 1 may utilize any available means of monitoring for changes in the expression level of the nucleic acids of the invention. As used herein, an active substance is said to modulate the expression of a nucleic acid of the invention, if it is capable of up-or down-regulating expression of the nucleic acid in a cell.

Substances that are assayed for in the above methods may be selected randomly or rationally or may be also designed. As used herein, a substance is said to be randomly selected when the substance is chosen randomly without considering the specific sequences involved in the association of the protein of the invention alone or with its associated substrates, binding partners, etc. An example of randomly selected substances is the use a chemical library or a peptide combinatorial library, or a growth broth of an organism.

The genes for the 5 ABC transporters identified as being differentially expressed in cells of interest may be used in a variety of nucleic acid detection assays to detect or quantify the expression level of a gene or multiple genes in a given sample. For example, traditional Northern blotting, nuclease protection, RT-PCR and differential display methods may be used for detecting gene expression levels.

The protein products of the genes identified herein can also be assayed to determine the amount of expression. Methods for assaying for a protein include Western blot, immunoprecipitation, radioimmunoassay and protein chips. Protein chips are supports bearing as capture probes antibodies or related proteins specific of the different proteins or peptides to be analyzed. Antibodies are either on the same support as a protein array or are on different supports as beads, each beads being specific for the detection of proteins. It is preferred, however, that the mRNA be assayed as an indication of expression.

Any hybridization assay format may be used, including solution-based and solid support-based assay formats. A preferred solid support is a low density array also known as a DNA chip or a gene chip. In one assay format, the array containing probes to at least 5 ABC transporter subfamilies as e.g. listed in table 1 may be used to directly monitor or detect changes in gene expression in cells or the treated cell as described herein. Assays of the invention may determine the expression level of about 14, 50, 100, 400, 1000 or even 3000 genes with some or all derived from table 1.

According to the present invention the number of genes to be detected may be limited since this allows to better focus on genes useful for the characterization multi-drug resistance and to give a more rapid and precise response to the questions of the prognostic, diagnostic and therapeutic follow up of the patients. The larger the number of genes to be analyzed and treated for data mining, the less efficient the correlation and the outcome of the analysis.

In another assay format, cells or cell lines are first identified, which express one or more of the gene products mentioned herein physiologically. Cells and/or cell lines thus identified will comprise the necessary cellular machinery to ensure that the transcriptional and/or translational apparatus mimics the response of sensitive or resistant tissue to the presence of a chemical substance. Such machinery would likely include appropriate surface transduction mechanisms and/or cytosolic factors. The tool as provided by this invention is then applied on these cells or cell lines and the invention is used as a research tool for investigating the role of the different ABC transporters in the tested experimental condition.

The present invention also pertains to a diagnostic and/or prognostic kit, which comprises means and media for performing the above method.

In another embodiment, the invention further envisages computer systems comprising a database containing information identifying the expression level in the tested cell or tissue of at least 5 genes associated with drug transport as listed in table 1 and a user interface to view the information. The database may further include sequence information for the genes, information identifying the expression level for the set of genes in reference cell or tissue and may contain links to external databases, such as GeneBank. The present invention includes relational databases containing sequence information, for instance for one or more of the genes of table 1, as well as gene expression information in various tissue samples. Databases may also contain information associated with a given sequence or tissue sample such as descriptive information about the gene associated with the sequence information, descriptive information concerning the clinical status of the tissue sample, or information concerning the patient from which the sample was derived. The database may be designed to include different parts, for instance a sequence database and a gene expression database. Methods for the configuration and construction of such databases are widely available, for instance from US-5,953,727, which document is incorporated herein by reference by way of reference.

The following examples illustrate the invention without limiting it thereto.

### Example 1:

Gene expression in leukemic cell lines CCRF-ADR5000 versus CCRF-CEM (Drug-resistant subline versus drug-sensitive parental cell line).

CCRF-ADR5000 is considered as the test and CCRF-CEM as the reference. The data are presented in the table 4 as ratio of test versus reference.

### 1. Leukemic Cell Lines:

ABCC1-overexpressing HL60/AR cell line and parental promyelocytic HL60 drug-sensitive cells (ABCC1-negative) were obtained from Dr. Sauerbrey (Jena, Germany), and seed in RPMI 1640 medium supplemented with 10 % fetal calf serum and 100 mM daunorubicin for HL60/AR. Control cell were seed followed the same schedule of medium changes without daunorubicin.

Human T-lymphoblastoid leukemic ABCB1-expressing CCRF-ADR5000 cells selected by Adriamycin and parental CCRF-CEM cell line (ABCB1-negative) were purchased from Dr Efferth (Heidelberg, Germany). Those cells were seed in RPMI 1640 medium with 10 % fetal calf serum. ABCG2-overexpressing MCF7/CH1000 cell line and parental human breast carcinoma MCF7 cell line (ABCG2-negative) were kindly provided by Dr Steinbach (Jena, Germany).

### 2. RNA extraction:

Poly (A)+ RNA was isolated from cell lines using the FastTrack 2.0 mRNA isolation Kit (Invitrogen) according to manufactures protocol for isolating mRNA starting from 4x10⁷ cells. To assess the integrity and relative contamination of mRNA with ribosomal RNA, analysis on bioanalyser (Agilent) were carried out.

The concentration and purity of RNA was determined by diluting an aliquot of the preparation in TE (10mM Tris-HCl pH 8, 1mM EDTA) and measuring (reading) its absorbance (in a spectrophotometer) at 260 nM and 280 nm. While A260 allows to evaluate the RNA concentration, the A260/A280 ratio gives an indication of RNA purity. For a RNA to be used, its ratio must be comprised between 1.8 and 2.

### 3. cDNA synthesis:

1 µl of poly(A+) RNA sample (0.5 µg/µl) was mixed with 2 µl oligo(dT)12-18 (0.5 µg/µl, Roche), 3.5 µl H20, and 2 µl of a solution of 6 different synthetic well-defined poly(A+) RNAs. These latter served as internal standards to assist in quantification and estimation of experimental variation introduced during the subsequent steps of analysis. After an incubation of 10 minutes at 70°C and 5 minutes on ice, 9 µl of reaction mix were added. Reaction mix consisted in 4 µl Reverse Transcription Buffer 5X (Gibco BRL), 1 µl RNAsin Ribonuclease Inhibitor (40 U/ml, Promega), and 2 µl of a 10X dNTP mix, made of dATP, dTTP, dGTP (5 mM each, Roche), dCTP (800 µM, Roche), and Biotin-11-dCTP (800 µM, NEN).

After 5 minutes at room temperature, 1.5 µl Superscript II (200 U/ml, Gibco BRL) was added and incubation was performed at 42°C for 90 minutes. Addition of Superscript and incubation were repeated once. The mixture was then placed at 70°C for 15 minutes and 1 µl Ribonuclease H (2U/µl) was added for 20 minutes at 37°C. Finally, a 3-minutes denaturation step was performed at 95°C. The biotinylated cDNA, was kept at -20°C.

### 4. Hybridization with biotinylated cDNA:

The array used in this study is composed of 42 transporter genes (including 39 ABC transporters, 1 cationic transporter (RAPT) and 2 ATP- sensitive potassium channels (Kir6.1, Kir6.2) and 8 housekeeping genes and is presented in figure 1. Several different controls including positive and negative detection control, positive and negative hybridization control, six different internal standards are all dispersed at different locations among the genes to be analyzed on the micro-array. In this example each spots was covered with a capture probe being a polynucleotide species which allow the specific binding of one target polynucleotide corresponding to a specific gene listed in figure 1.

Hybridization chambers were from Biozym (Landgraaf, The Netherlands). Hybridization mixture consisted in biotinylated cDNA (the total amount of labeled cDNA), 6.5 µl HybriBuffer A (Eppendorf, Hambourg, Germany), 26 µl HybriBuffer B ( Eppendorf, Hambourg, Germany), 8 µl H2O, and 2 µl of positive hybridization control.

Hybridization was carried out overnight at 60°C. The micro-arrays were then washed 4 times for 2 min with washing buffer (B1 0.1X + Tween 0.1 %) (Eppendorf, Hamburg, Germany).

The micro-arrays were than incubated for 45 minutes at room temperature with the Cy3-conjugated IgG Anti biotin (Jackson Immuno Research Laboratories, Inc #200-162-096) diluted 1/1000 X Conjugate-Cy3 in the blocking reagent and protect from light.

The micro-arrays were washed again 5 times for 2 minutes with washing buffer (B 1 0.1X + Tween 0.1 %) and 2 times for 2 minutes with distilled water before being dried under a flux of N2.

### 5. Scanning and data analysis:

The hybridized micro-arrays were scanned using a laser confocal scanner "ScanArray" (Packard, USA) at a resolution of 10 µm. To maximize the dynamic range of the assay the same arrays were scanned at different photomultiplier tube (PMT) settings. After image acquisition, the scanned 16-bit images were imported to the software, 'ImaGene4.0' (BioDiscovery, Los Angeles, CA, USA), which was used to quantify the signal intensities. Data mining and determination of significantly expressed gene in the test compared to the reference arrays was performed according to the method described by Delongueville et al (Biochem Pharmacol. **64**(1) (2002), 137-49). Briefly, the spots intensities were first corrected for the local background and than the ratio between the test and the reference arrays were calculated. To account variation in the different experimental steps, the data obtained from different hybridizations were normalized in two ways. First the values are corrected using a factor calculated from the intensity ratios of the internal standard reference and the test sample. The presence of 6 internal standard probes at different locations on the micro-array allows measurement of a local background and evaluation of the micro-array homogeneity, which is going to be considered in the normalization (Schuchhardt et al., Nucleic Acids Res. **28** (2000), E47). However, the internal standard control does not account for the quality of the mRNA samples, therefore a second step of normalization was performed based on the expression levels of housekeeping genes. This process involves calculating the average intensity for a set of housekeeping genes, the expression of which is not expected to vary significantly. The variance of the normalized set of housekeeping genes is used to generate an estimate of expected variance, leading to a predicted confidence interval for testing the significance of the ratios obtained (Chen et al, J. Biomed. Optics **2** 1997, 364-74). Ratios outside the 95% confidence interval were determined to be significantly changed by the treatment.

The reliability and the reproducibility of the assay is assessed by repeating the experiments three times for each cell line (n=3). The fluorescence intensity ratios for the test versus the reference are presented in table 4. The results are presented as the mean of the ratios and the standard deviation of three different experiments. The results are significant changes in gene expression between the two samples. CCRF-CEM is considered as the reference and CCRF-ADR5000 as the test. A clear overexpression of the ABCB1 gene in the resistant cell line as compared to the reference could be observed.

### Example 2:

Gene expression in leukemic cell lines HL60 A/R versus HL60 Sens (Drug-resistant subline versus drug-sensitive parental cell line).

HL60 A/R is considered as the test and HL60 Sens as the reference. The experiment was performed as described in the example 1. The 4 steps were RNA extraction, cDNA synthesis, hybridization on the array and the quantification and analysis of the results. The results are presented as the mean of the ratios and the standard deviation of three different experiments. The data are presented in the table 4 as ratio of test versus reference. An over-expression of the ABCC1 gene in the resistant cell line as compared to the reference could be observed.

### Example 3:

Gene expression in leukemic cell lines MCF7-CH1000 versus MCF7 parental (Drug-resistant subline versus drug-sensitive parental cell line).

MCF7-CH1000 is considered as the test and MCF7 parental as the reference. The experiment was performed as described in the example 1. The results are presented as the mean of the ratios and the standard deviation of three different experiments. The data are presented in the table 4 as ratio of test versus reference. An over-expression of the ABCG2 gene in the resistant cell line as compared to the reference could be observed.

### Example 4:

Basal gene expression of ABC genes in sensitive leukemic cell lines CCRF-CEM, HL60 Sens and MCF7 parental.

Data are those of example 1, 2 and 3. The data are presented in the table 5 as the absolute values for the sensitive cell lines as reported to the housekeeping genes value fixed at 100%. In the CCRF-CEM and HL60 Sens cell lines, the values are compared to the housekeeping gene HPRT and in the MCF7 parental they are compared to the housekeeping gene alphatubulin. The data are processed as follows. The spots intensities are first corrected for the local background and the average values of the spots replicates are calculated for each experiment. The values obtained are reported to the mean value of the considered housekeeping gene in the three experiments and multiplied by 100. Finally, the mean values of the three experiments are calculated and reported in Table 5.

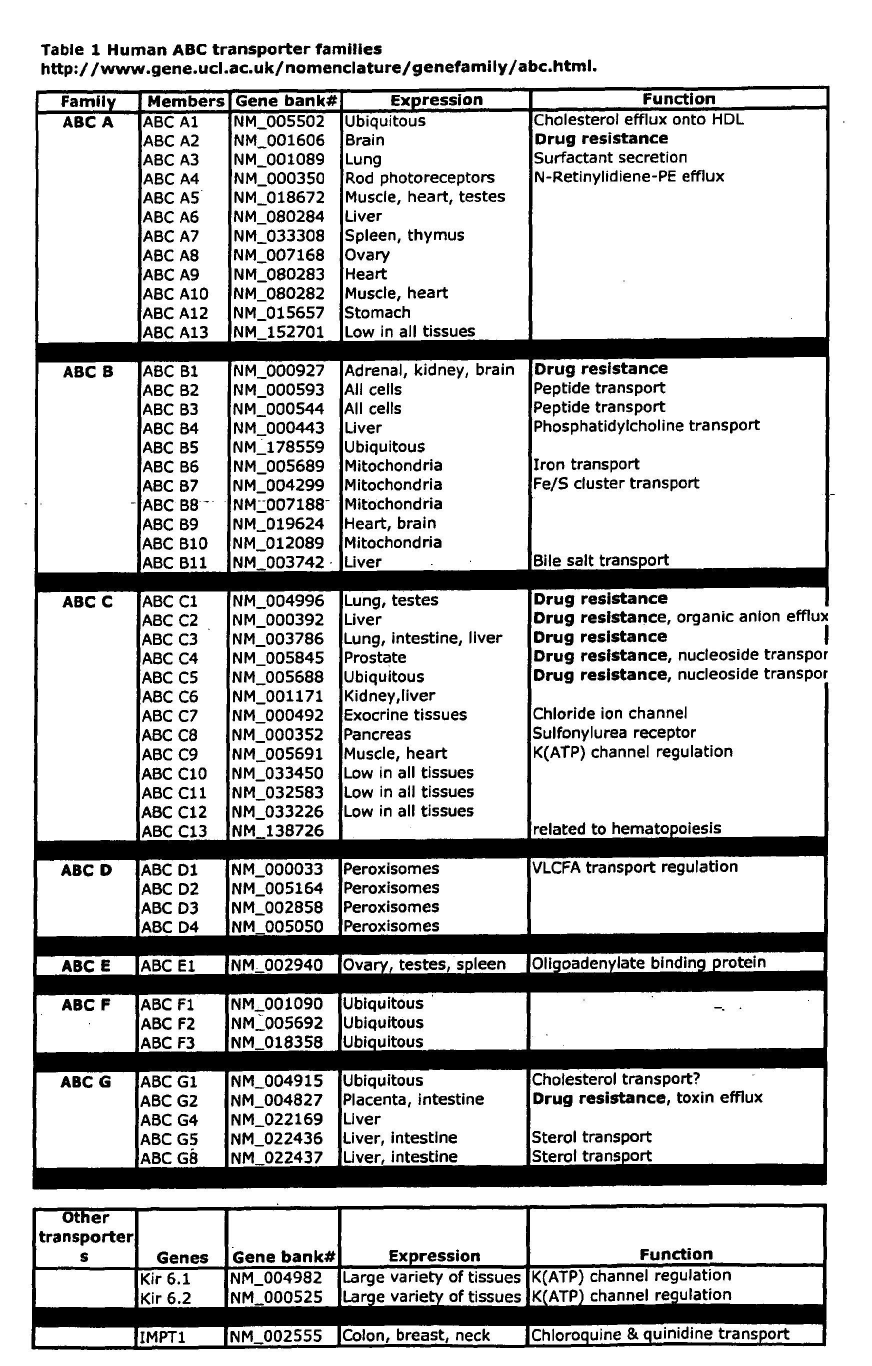

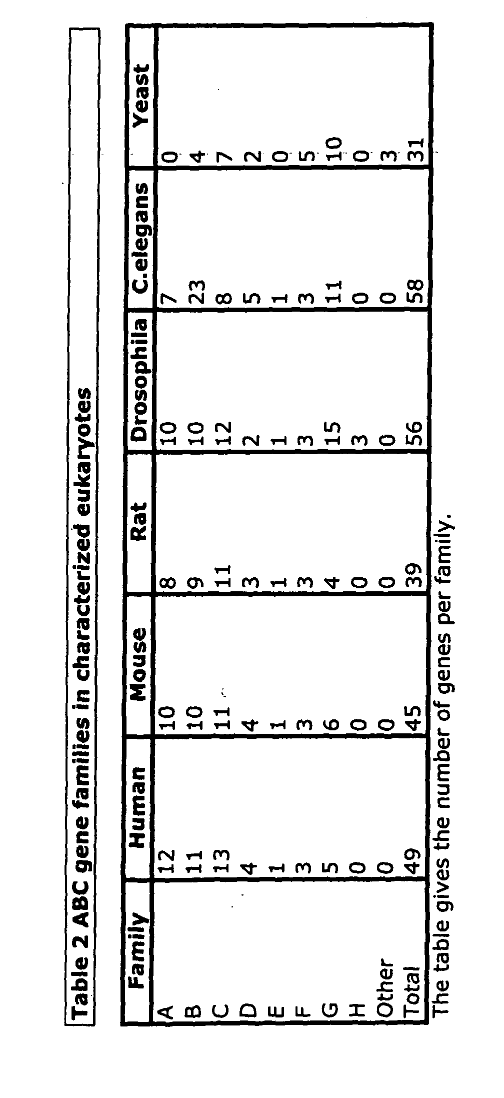

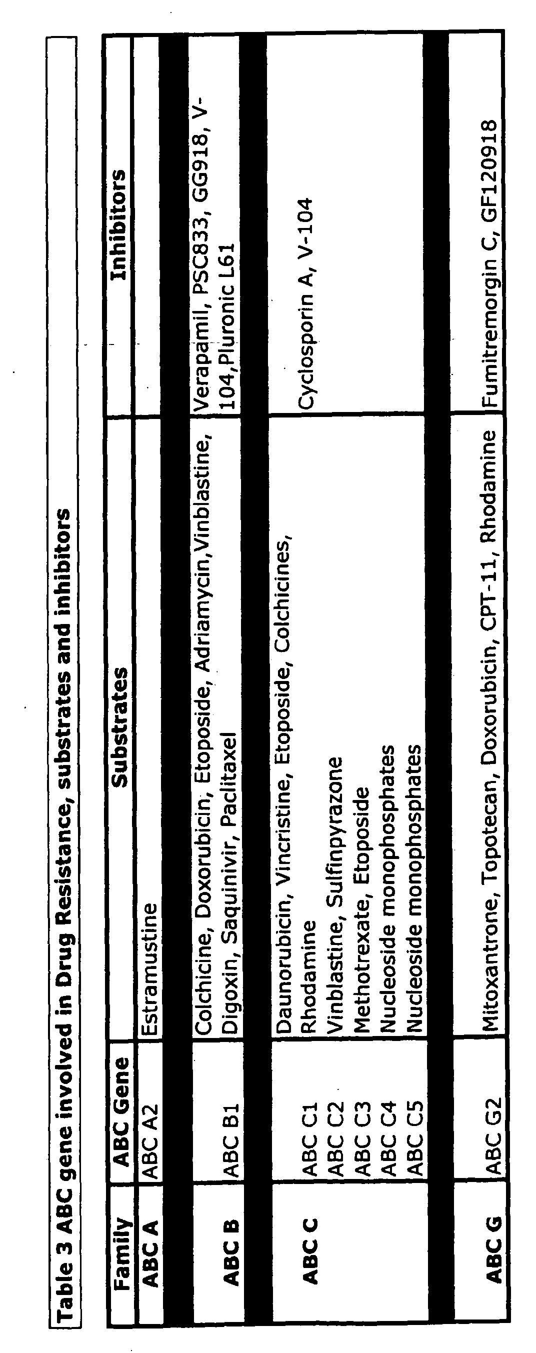

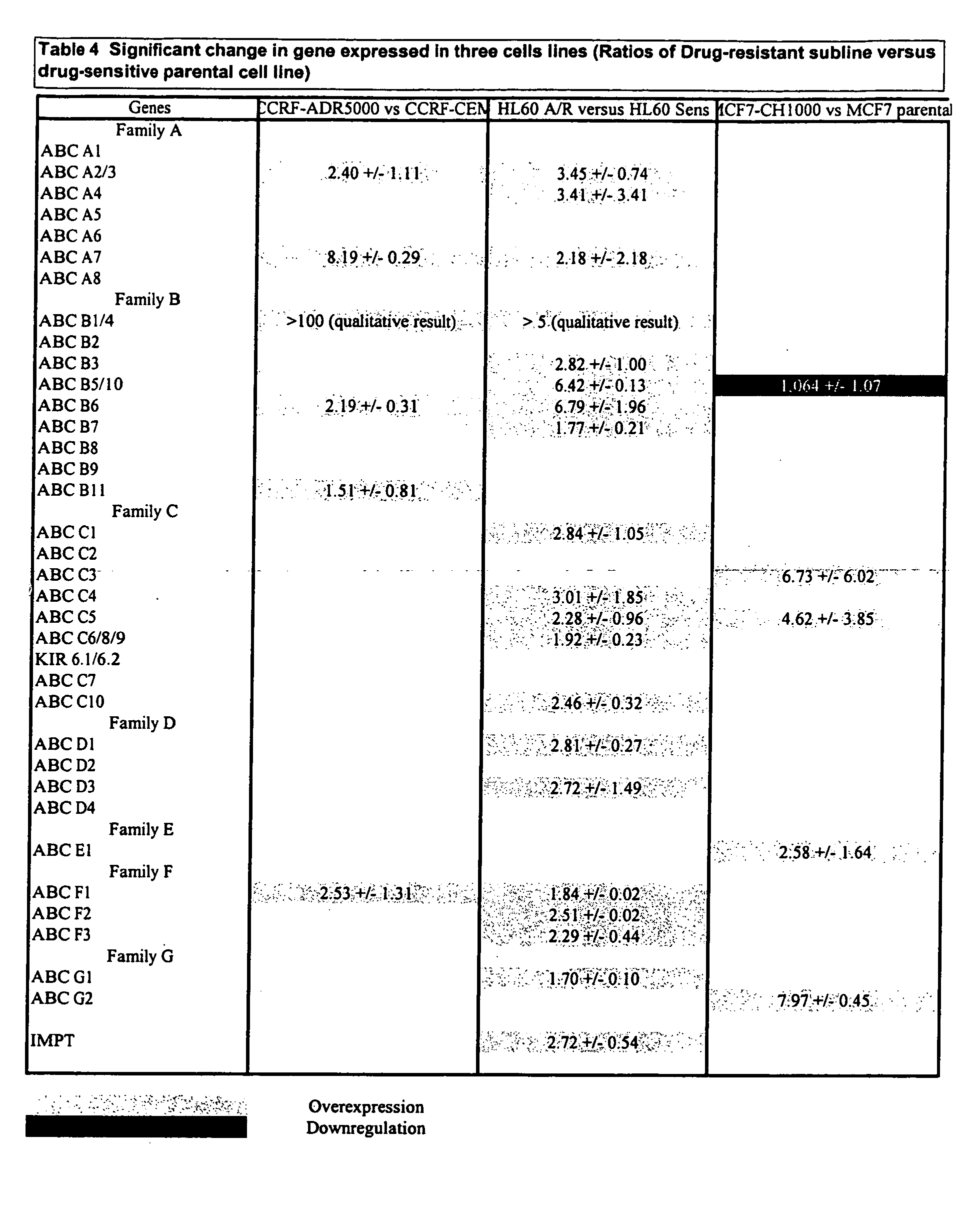

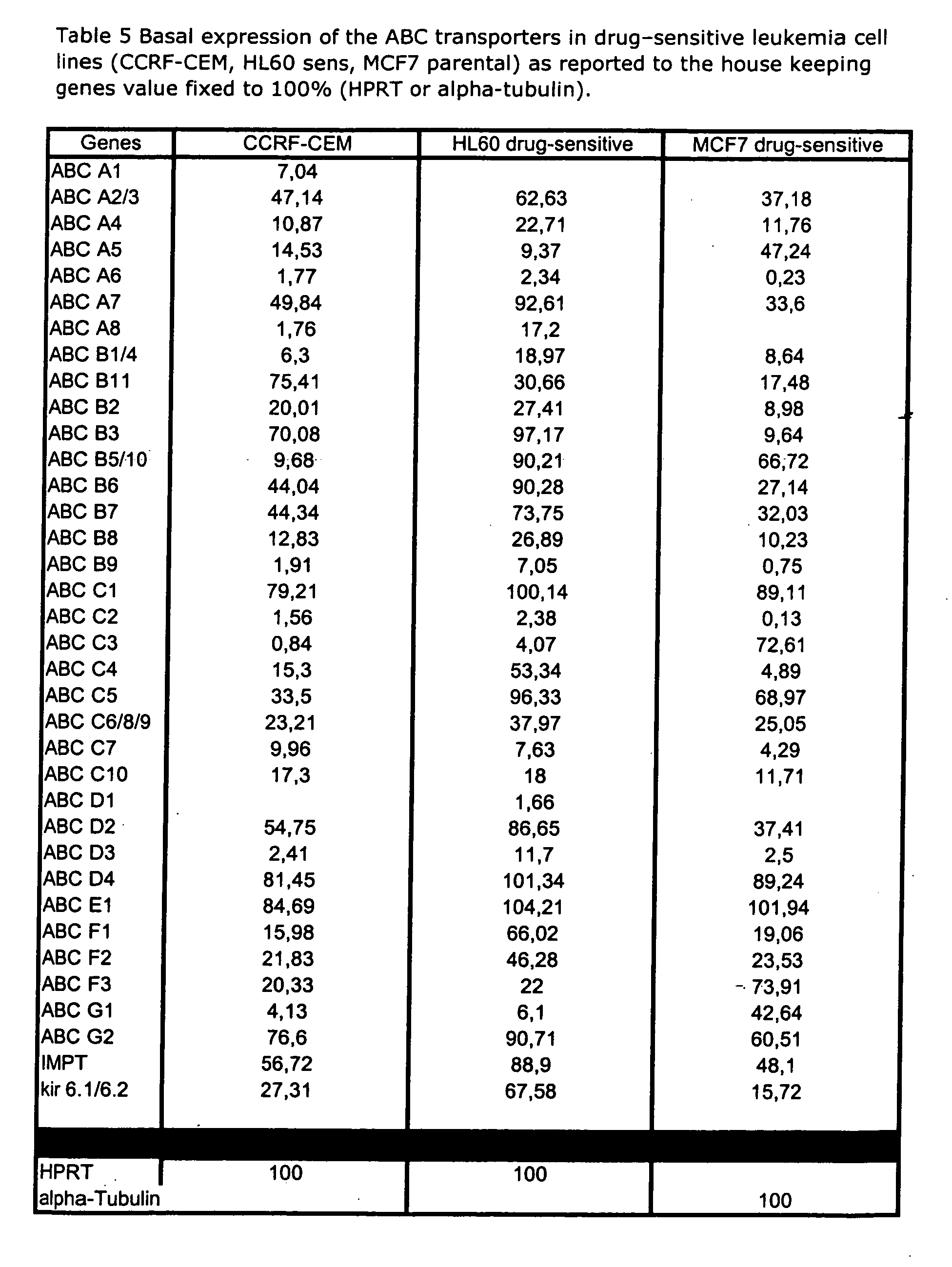

## Claims

1. A method for the determination of the resistance of cells versus the action of an active substance comprising the steps of:
(i) providing a sample containing cells exposed or having been exposed to an active substance,
(ii) analyzing the gene expression pattern of said cells on a micro-array, containing on specific locations thereon capture probes for the specific detection and quantification of at least 5 ABC transporters,
wherein a change of the expression of at least 5 ABC transporters by a factor of at least about 1.5 as compared to a reference is indicative of the development and/or existence of resistance of said cells to the substance.

2. The method of claim 1 for the determination of gene expression pattern for at least 5, 10, 39 and 49 ABC transporters selected from those listed in Table 1.

3. The method of claim 1 for the determination of gene expression pattern for at least 5 genes of the ABC transporter family having unravelled multi-drug resistance function as provided in Table 1.

4. The method of claims 1-3 for correlating the resistance of cells from a patient to the chemotherapy by a given drug.

5. The method of claims 1-3 for the determination of the resistance of a cell to a drug selected from Table 3.

6. The method of claims 1-5 for the determination of the gene expression changes in cells associated with the drug resistance of cells incubated in the presence of the drug

7. The method of claim 6 wherein the cells are derived from a patient and method is designed the determination of a potential active drug for the patient treatment.

8. The method of claims 1-3 for the determination of the activity of a drug against an analysed cell.

9. The method of claims 1-3 for the selection of an active drug for patient treatment.

10. The method of claims 1-3 for monitoring a patient treated with a drug for chemotherapy.

11. The method of any of the preceding claims, wherein the micro-array contains at least one gene selected from Kir6.1, Kir6.2 and IMPT.

12. The method of any of the preceding claims for the screening of ABC transporters in acute myeloid leukemia.

13. The method of any of the preceding claims for the screening of ABC transporters in acute lymphocytic leukemia.

14. The method of any of the preceding claims for the screening of ABC transporters in solid tumors.

15. The method of any of the preceding claims in which the capture probes are single stranded nucleotides

16. The method of any of the preceding claims in which one location give the quantification of one ABC transporters gene.

17. A kit containing an array with capture probes located at specific locations for the detection and quantification of at least 5 ABC transporters of the gene expression of ABC transporters as provide by one of the preceding claims.
